# EUROPEAN PATENT SPECIFICATION

(11) **EP 2 000 085 B1**
(45) Date of publication and mention of the grant of the patent: **27.03.2013**
(21) Application number: 08157687.8
(22) Date of filing: 05.06.2008
(51) Int. Cl.: A61B 5/0432, A61B 5/0404

(54) **Portable electrocardiographic apparatus**
Tragbares EKG-Gerät
Appareil ECG portable

(30) Priority: 07.06.2007 IT MO20070194
(43) Date of publication of application: 10.12.2008
(73) Proprietor: Politecnico di Milano, 20133 Milano (IT)
(72) Inventor: Costantino, Maria Laura, 20090 Segrate (MI) (IT); Di Mattia, Daniele Giorgio Edoardo, 20122 Milano (IT); Fincati, Fabio, 20153 Milano (IT)
(74) Representative: Crugnola, Pietro

(56) References cited:
- DE-A1- 10 233 071
- US-A1- 2005 014 999
- US-A1- 2006 217 620
- US-A1- 2007 055 166

## Description

This invention relates to apparatuses for measuring and recording the electrical activity of the heart, in particular, a portable electrocardiographic apparatus suitable for detecting and recording an electric signal relative to an electrocardiographic (ECG) tracing or a trace of the heart.

The problem of detecting and recording the electrical activity of the cardiac muscle of a subject at the time of occurrence of a subjective sensation of arrhythmia or other cardiac anomaly, for check the actual appearance and the features is known. These anomalies occur, in fact, suddenly and unexpectedly, and have a variable duration, generally relatively brief and therefore difficult to detect and measure during a specialist medical check-up. Moreover, with traditional electrocardiographic apparatuses, it is necessary to carry out a series of preparatory operations for recording the electric signal, which can delay correct detection of the cardiac anomaly.

It is, therefore necessary to have portable, light and compact apparatuses which can be easily and rapidly used anywhere by a user to check his own cardiac activity or that of other individuals.

Portable cardio frequency meters are known and widely used, in the form of bracelets or watches to be worn on the wrist or bands to be applied on the chest, having such limited weight and dimensions so as to allow their use also during physical activity.

These apparatuses however allow the user to measure and display only his/her own cardiac frequency, i.e. the number of heartbeats per minute. Some apparatuses can store data relative to the frequency and download these data to a computer by means of a suitable connection.

The portable cardio frequency meters are therefore not capable of checking and measuring the electrical activity of the heart and detecting cardiac anomalies, if any, with precision.

Portable electrocardiographs are known capable of measuring and recording the electric signal relative to the ECG tracing of the cardiac muscle, which integrate in a compact and limited sized unit the electronic circuits for the acquisition and processing of electrical signals relative to the cardiac activity, a graphic screen for displaying the ECG tracing and generally a printer for printing said ECG tracing. The electric signals are detected by electrodes applied to the subject being examined and connected by means of wires or cables to the device. The latter is powered by rechargeable batteries and can also be connected to an external computer for downloading the data stored.

Even if the dimensions and the weight of these apparatuses are relatively limited - approximately having the dimensions and the weight of a portable computer - therefore such as to allow the apparatuses to be easily transported and used, they cannot however be worn or, in any case, carried continuously by a user during daily activities for measuring possible and sudden cardiac anomalies.

Further portable electrocardiographs exist which can be worn for continuous measurement of an ECG tracing, by means of electrodes conveniently applied on the body and connected to the apparatus by means of wires. Such apparatuses have limited dimensions and weight and record the data on a magnetic and electronic support from which they can be subsequently downloaded and/or transmitted to a fixed electrocardiographic unit, arranged for processing, displaying, printing and storing said data.

A drawback of these apparatuses lies in the fact that these apparatuses can only be used by specialist medical personnel, necessary for positioning the electrodes correctly on the subject to be monitored and for activating, checking and controlling the working of the apparatus.

For this reason, the aforementioned portable electrocardiographs are not suitable for sudden and temporary use, i.e. for measuring the electrocardiographic trace if necessary, as they are for specialist use for monitoring the electrical activity of the cardiac muscle continuously for medium/long periods, typically hours and/or days.

Moreover, the portable apparatuses and the relative fixed unit are quite complex and expensive.

US 2007/055166 A1 discloses a system comprising a plurality of biometric sensors to be affixed to a patient. The system provides for transmitting analog signals from the biometric sensors to a self-contained module wherein the signal is conditioned, digitalized, stored and transmitted to a local host that in turn transmits the data to a remote host. The module is provided with data storage means that may be removable. The biometric sensors comprise one ECG electrode attached to the left arm of a patient and one ECG electrode attached to the right arm of a patient. The ECG electrodes are thin disks comprising a metal stud to detect the electric potential at the attachment site. A left arm component and a right arm component are mounted directly onto the electrode studs and linked by a flexible cable. The left arm component comprises a coin-type battery and the right arm component is comprised of a micro-controller an amplifier (16) and a radiofrequency transmitter.

US 2006/217620 A1 discloses a device for cordless recording, telecommunication transmission and processing of three special ECG leads by means of a mobile device. The mobile device comprises five integrated electrodes, two of which are located on the front side of the device and three are on the back side of the device. Each of the electrodes is connected in such a way as to record a different potential.

DE 10233071 A1 discloses a mobile phone provided with four electrodes on the back of the mobile phone for measuring EKG signals which can be transmitted to a remote center.

US 2005/014999 discloses a compact diagnostic device for the examination and monitoring of vital parameters of the human body, comprising four measuring electrodes provided on the bottom of a casing, at least one of which being removable by means of a connection cable to be positioned on an area of the body to be examined on its own.

An object of this invention is to improve the known portable apparatuses for measuring and recording an electric signal relative to an electrocardiographic (ECG) trace of the heart of a user.

Another object is to make a portable electrocardiographic apparatus which will make it possible to store and/or easily, rapidly and economically transfer the data detected during working to any personal computer or electronic processor for being processed, read and evaluated by medical personnel.

A further object is to obtain an apparatus which can be used intuitively and immediately by any person, even not expert, without the need to carry out complex and long preparatory procedures for measurement.

Another object is to obtain an apparatus which makes it possible to precisely and accurately measure and record an electric signal relative to an ECG trace with different electrocardiographic leads, in particular standard and/or precordial leads.

A further object is to make a pocket-sized apparatus, i.e. with dimensions and weight so as to allow an user to carry it easily everywhere and every time, to be used at once when necessary.

A still further object is to make a sturdy and reliable apparatus having a simple and economic manufacture.

According to the invention, a portable apparatus is provided for measuring and storing an electric signal relative to an electrocardiographic trace comprising electrode means for interfacing portions of the body of a user to detect an electric signal relative to an electrocardiographic trace of the latter, processing means for acquiring said electric signal by said electrode means and memory means for storing said electric signal acquired by said processing means, said memory means being removable, support means having two side walls arranged for containing said processing means (5) and said memory means (6), said electrode means comprising a first electrode (3) and a second electrode (4) connected to said processing means (5) and fixed to said support means (2) and inserted in a portion of said support means (2) so that each of these electrodes is contactable by at least one finger of a respective hand of said user, characterized in that said first electrode (3) and said second electrode (4) are so shaped and arranged as to have respective free interface surf aces on both the side walls of said support means (2). Owing to this aspect of the invention, it is therefore possible to make a portable electrocardiographic apparatus which, by storing the data detected during working on removable memory means, transfers the aforementioned data easily and rapidly to any computer or external electronic processor so that they can be processed, read, evaluated by medical personnel.

The memory means comprises, in particular, a known type of electronic memory card, having non volatile flash memory, with limited cost, which can be easily read by a personal computer. Alternatively, the memory means may comprise a "SIM Card" i.e. a programmable card used in mobile phones to allow the mobile phones to be connected to the mobile telephone network. When the SIM Card, which is provided with flash memory, is removed from the apparatus of the invention and inserted in a mobile phone, the data acquired can be transmitted to a remote services centre for their evaluation, through a mobile telephone network.

The apparatus comprises support means inside which the processing means and memory means are arranged and to which the electrode means is fixed. The support means comprises a containing body or a containing frame made of plastic, flat shaped, for example rectangular, pocket-sized, almost like a standard electronic card or a credit card.

In virtue of the pocket size and reduced weight, a user can always carry the electrocardiographic apparatus of the invention and be capable of immediately measuring and recording, with precision, the electrical activity of his/her own cardiac muscle or that of any person, when a subjective sensation of arrhythmia or other sensation of anomalous cardiac function is felt.

Owing to the invention, it is possible to make a portable electrocardiographic apparatus which can be used intuitively and immediately by any person, even not expert, without the need to carry out complex and long preparatory procedures for measurement. Since the electrodes are fixed to a lateral portion of the support means, to measure and record the electric signal of an ECG trace in D1 standard lead, it is sufficient for the user to position at least one finger of each hand on said electrodes, so as to close the circuit and to allow the processing means to receive and record said electric signal.

The activation time of the apparatus and measuring methods by means of contact of the electrodes with the fingers of the hands allow the electric signal to be detected and stored in a very short time, without the need for carrying out operations or procedures which could delay correct detection of a possible cardiac anomaly.

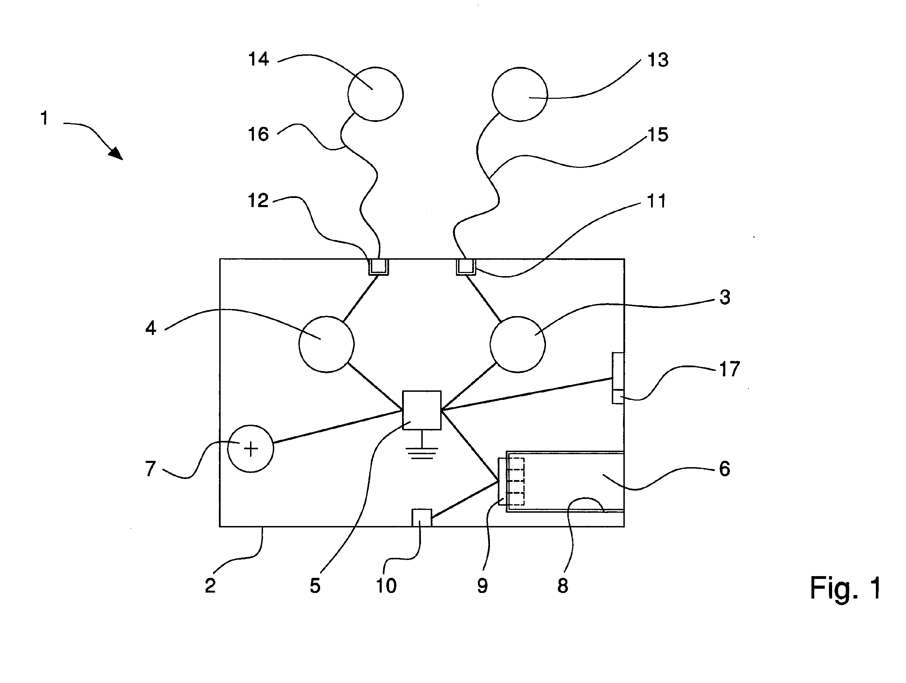

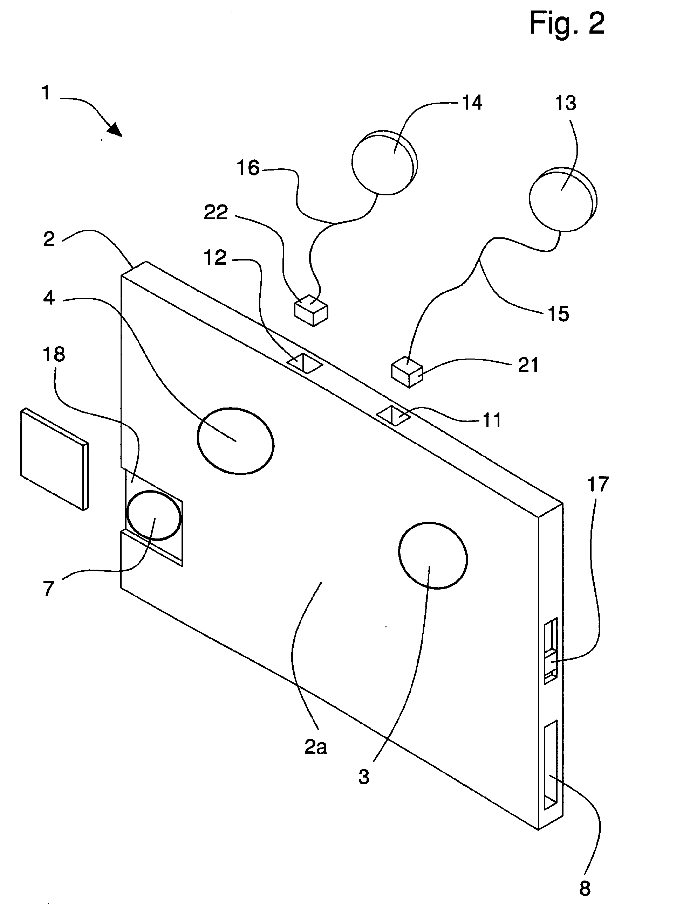

The apparatus further comprises further electrodes which can be arranged by the user on his/her own chest or that of another person to measure and record precordial ECG leads. The invention will be better understood and carried into effect with reference to the attached drawings, in which an embodiment is shown by way of non-limiting example, in which: Figure 1 is a schematic view of the portable electrocardiographic apparatus of the invention; Figure 2 is a schematic axonometric view of the apparatus in Figure 1.

With reference to Figures 1 and 2, a portable apparatus 1 is shown for measuring an electric signal relative to an electrocardiographic (ECG) trace comprising support means 2 inside which electrode means 3, 4 is arranged for interfacing distinct portions of a body of a user and detecting an electric signal, processing means 5 for acquiring said electric signal by said electrode means 3, 4 and memory means 6 of removable type arranged for storing the electric signal acquired by processing means 5.

Interface means 9 is also provided which allows the processing means 5 to be electrically connected to the memory means 6.

Battery means 7 is housed inside the support means 2 to electrically power to the transducer means 5 and the memory means 6.

The support means 2 comprises a containing body or a containing frame made of plastic and having a flat shape, substantially rectangular, with a size substantially like that of a standard electronic card or a credit card, i.e. having a length comprised, for example, between 70 and 100 mm, in particular 85 mm, a width comprised between 40 and 70, in particular 55 mm, and a thickness comprised between 3 and 15 mm, in particular 5 mm.

These dimensions allow the apparatus 1 to be put easily in a pocket of a user.

The electrode means comprises a first electrode 3 and a second electrode 4 suitably spaced and inserted in at least a side wall 2a of the support means 2 so as to maintain a respective interface surface free for contact with the body of the user.

Furthermore, electrodes 3, 4 are so shaped and arranged as to have respective free interface surfaces on both the side walls of the support means 2.

The two electrodes 3, 4 are connected electrically to the processing means 5 which comprises a known type of electronic processing unit, capable of acquiring the analog electric signal relative to the ECG trace.

In particular, the processing means 5 is arranged for digitalizing that is converting the continuous analog electric signal measured by the electrodes 3, 4, having voltage, for example, comprised between -40mV and +40mV, into a discrete digital signal which can be recorded on the memory means 6 in the form of data files in text or ASCII format. The processing means 5 acquires the analog electric signal with a suitable sampling frequency, such as to allow the original signal to be reproduced correctly, for example a frequency not less than 1000 samples/second.

The processing means 5 also comprises an internal clock to mark and identify each data file with the exact date and time of the measurement.

The memory means 6 is of the removable type and comprises a memory card, i.e. an electronic card provided with flash memory capable of storing the data in digital form and retaining the data in memory even in the absence of electricity (non volatile memory).

This memory card 6 may be any one of the multiple types of commercially available cards, having different sizes, capacities and technical features, for example, CompactFlash, SmartMedia, Memory Stick, etc.

Alternatively, the memory means 6 may comprise a "SIM Card" i.e. a programmable card used in mobile phones to allow the mobile phones to be connected to the mobile telephone network. The SIM Cards are, in fact, provided with flash memory, variable from 8 to 256 Kbytes. As explained in greater detail in the course of the description, the use of a SIM Card as means of memory allows to transmit the data obtained by the apparatus 1 during the working by means of a mobile phone supporting said SIM Card.

In a further embodiment of the apparatus 1 not shown, memory means 6 comprises a USB key i.e. a portable mass storage unit that can be used by means of a USB port of a common personal computer.

The support means 2 comprises a seat 8 suitable for removably housing the memory card 6 and provided with interface means 9. The seat 8 and the interface means 9 are configured and arranged on the basis of the type of memory card 6 used.

The seat 8 comprises, for example, a slit made in the thickness of the support means 2, having length equal to that of the memory card 6 and provided with an end opposite the insertion opening of the interface means 9.

If the memory means 6 comprises a USB key, the interface means 9 includes a USB port.

The apparatus 1 further comprises connecting means 10 suitable for connecting the memory card 6, by means of interface means 9, to an external electronic processor or personal computer. The connecting means 10 comprises, for example, a serial connector, in particular, a mini USB connector.

Linking means 11, 12 allows electrodes 4, 3 to be connected to respective cables 15, 16 at the ends of which respective further electrodes 13, 14 are placed, which can be positioned on the chest of a user to allow the apparatus 1 to measure and record electric signals relative to precordial ECG leads. The linking means comprises a pair of known type of respective interlocking connectors 11, 12, not shown in detail, to which complementary connectors 21, 22 provided at the free ends of cables 15, 16 can be hooked removably.

In an embodiment of the apparatus not shown, the linking means 11, 12 is directly connected to the processing means 5. The battery means 6 is of removable type, contained in a further seat 18 of the support means 2.

The battery means 6 may be also of rechargeable type, for example, by means of an external power source connectable to said battery means 6 by the connecting means 10.

The apparatus 1 comprises switch means 17 arranged for activating or deactivating the processing means 5.

The working of the portable electrocardiographic apparatus 1 of the invention provides that a user, after switching on the apparatus 1 by activating the switch means 17, positions at least one finger of each hand on electrodes 3, 4 so as to close the circuit and allow the processing means 5 to receive and record an electric signal relative to an ECG lead trace, in particular, a trace with DI standard lead features.

This continuous analog electric signal forms an ECG tracing which is sampled by the processing means 5 and converted into a digital signal which can be stored in the form of file in ASCII format on the memory card 6.

The sampled signal is stored in memory as long as the user keeps the circuit closed with electrodes 3, 4 or switches off the apparatus using the switch 17.

It is advisable to underline the fact that the activation time of the apparatus and measuring methods by means of contact of the electrodes 3, 4 with the fingers of the hands allow the electric signal to be detected and stored in a very short time, without the need for carrying out preparatory operations for recording the signal, which could delay correct detection of a possible cardiac anomaly.

Once one or more files relative to the respective ECG traces measured and processed have been stored, the user can easily remove the memory card 6 from the seat 8 of the support means 2 to allow the data to be transferred to an external computer provided with suitable reading means for reading said memory card.

The recorded data can be displayed and studied by medical personnel to identify and determine possible cardiac pathologies or heart troubles.

If the memory means 6 comprises a SIM card, the latter can be inserted in a mobile phone and transmitted by connection to the mobile telephone network, for example the GSM network, to a services centre in which said data are processed and evaluated by medical personnel.

The apparatus 1 also allows an electric signal relative to an ECG trace in precordial leads to be easily measured and recorded. For this purpose, it is enough to connect the further electrodes 13, 14 to respective interlocking connectors 11, 12 of the support means 2 and apply said further electrodes 13, 14 on the chest of the subject to be monitored. Once the apparatus 1 is switched on by means of the switch 17, the processing means 5 starts sampling and recording the ECG tracing on the memory card 6.

It is advisable to underline that, in virtue of the pocket size and reduced weight, a user can always carry the electrocardiographic apparatus of the invention and be thus capable of immediately measuring and recording, with precision, the electrical activity of his/her own cardiac muscle or that of any person when a subjective sensation of arrhythmia or other sensation of anomalous cardiac function is felt, to check the actual occurrence and the features.

The sturdy structure of the apparatus 1 and the simple and immediate working allow the apparatus to be used in any situation by anyone not necessarily expert or particularly capable.

## Claims

1. Portable apparatus for measuring and storing an electric signal relative to an electrocardiographic trace, comprising electrode means (3, 4) for interfacing portions of the body of a user to detect an electric signal relative to an electrocardiographic trace of the latter, processing means (5) for acquiring said electric signal by said electrode means (3, 4), memory means (6) for storing said electric signal acquired by said processing means (5), said memory means (6) being removable, support means having two side walls (2a) two side walls (2a) and arranged for containing said processing means (5) and said memory means (6), said electrode means comprising a first electrode (3) and a second electrode (4) connected to said processing means (5) and fixed to said support means (2) and inserted in a portion of said support means (2) so that each of these electrodes is contactable by at least one finger of a respective hand of said user, **characterized in that** said first electrode (3) and said second electrode (4) are so shaped and arranged as to have respective free interface surfaces on both the side walls of said support means (2).

2. Apparatus according to claim 1, wherein said memory means (6) comprises at least one non volatile mass memory electronic card.

3. Apparatus according to claim 1, wherein said memory means (6) comprises a programmable electronic "SIM Card" associable with a mobile phone for connection to a mobile telephone network.

4. Apparatus according to claim 1, wherein said memory means (6) comprises a portable mass storage unit usable by means of a USB port.

5. Apparatus according to any one of the preceding claims, comprising interface means (9) for connecting said processing means (5) to said memory means (6).

6. Apparatus according to any one of the preceding claims, comprising connecting means (10) for connecting said memory means (6) and/or said processing means (5) to external electronic processing means, in particular, a personal computer.

7. Apparatus according to any one of the preceding claims, comprising further electrode means (13, 14) provided with cable means (16, 15) for removable connection to said electrode means (3, 4) and/or said processing means (5).

8. Apparatus according to claim 7, comprising linking means (11, 12) connected to said electrode means (3, 4) and/or to said processing means (5) and arranged for receiving respective complementary connectors (21, 22) of said cable means (15, 16).

9. Apparatus according to any one of claims 1 to 8, wherein said containing body or said containing frame is pocket-sized and substantially rectangular shaped, with length comprised between 70 and 100 mm, in particular 85 mm, width comprised between 40 and 70, in particular 55 mm, and thickness comprised between 3 and 15 mm, in particular 5 mm.

10. Apparatus according to any one of the preceding claims, comprising battery means (7) for powering at least said processing means (5) and said memory means (6).

11. Apparatus according to claim 10, wherein said battery means is removable.

12. Apparatus according to any one of the preceding claims, comprising switch means (17) for activating or deactivating at least said processing means (5).

13. Apparatus according to any one of the preceding claims, wherein said processing means (5) comprises an electronic unit arranged for converting said electric signal of analog type, detected by said electrode means (3, 4) into a discrete digital signal which can be recorded in the form of data file on said memory means (6).

14. Apparatus according to any one of the preceding claims, wherein said processing means (5) is arranged for acquiring said electric signal with a predefined sampling frequency, in particular, a frequency not less than 1000 samples per second.

15. Apparatus according to any one of the preceding claims, and suitable for measuring and recording the electric signal of an ECG trace in D1 standard lead.

## Patentansprüche

1. Tragbare Vorrichtung zum Messen und Speichern eines elektrischen Signals relativ zu einer elektrokardiographischen Kurve mit einer Elektrodeneinrichtung (3, 4) zum Koppeln von Abschnitten des Körpers eines Benutzers, um ein elektrisches Signal relativ zu einer elektrokardiographischen Kurve des letzteren zu detektieren, einer Verarbeitungseinrichtung (5) zum Erfassen des elektrischen Signals durch die Elektrodeneinrichtung (3, 4), einer Speichereinrichtung (6) zum Speichern des durch die Verarbeitungseinrichtung (5) erfassten elektrischen Signals, wobei die Speichereinrichtung (6) entnehmbar ist, einer Trägereinrichtung mit zwei Seitenwänden (2a), die zum Enthalten der Verarbeitungseinrichtung (5) und der Speichereinrichtung (6) angeordnet ist, wobei die Elektrodeneinrichtung eine erste Elektrode (3) und eine zweite Elektrode (4) umfasst, die mit der Verarbeitungseinrichtung (5) verbunden sind und an der Trägereinrichtung (2) befestigt sind und in einen Abschnitt der Trägereinrichtung (2) eingefügt sind, so dass jede von diesen Elektroden durch mindestens einen Finger einer jeweiligen Hand des Benutzers kontaktierbar ist, **dadurch gekennzeichnet, dass** die erste Elektrode (3) und die zweite Elektrode (4) so geformt und angeordnet sind, dass sie jeweilige freie Schnittstellenoberflächen an beiden Seitenwänden der Trägereinrichtung (2) aufweisen.

2. Vorrichtung nach Anspruch 1, wobei die Speichereinrichtung (6) mindestens eine nichtflüchtige elektronische Massenspeicherkarte umfasst.

3. Vorrichtung nach Anspruch 1, wobei die Speichereinrichtung (6) eine programmierbare elektronische "SIM-Karte" umfasst, die mit einem Mobiltelefon zur Verbindung mit einem Mobiltelefonnetz verbindbar ist.

4. Vorrichtung nach Anspruch 1, wobei die Speichereinrichtung (6) eine tragbare Massenspeichereinheit umfasst, die mittels eines USB-Anschlusses verwendbar ist.

5. Vorrichtung nach einem der vorangehenden Ansprüche mit einer Schnittstelleneinrichtung (9) zum Verbinden der Verarbeitungseinrichtung (5) mit der Speichereinrichtung (6).

6. Vorrichtung nach einem der vorangehenden Ansprüche mit einer Verbindungseinrichtung (10) zum Verbinden der Speichereinrichtung (6) und/oder der Verarbeitungseinrichtung (5) mit einer externen elektronischen Verarbeitungseinrichtung, insbesondere einem Personalcomputer.

7. Vorrichtung nach einem der vorangehenden Ansprüche, die ferner eine Elektrodeneinrichtung (13, 14) umfasst, die mit einer Kabeleinrichtung (16, 15) zur entfernbaren Verbindung mit der Elektrodeneinrichtung (3, 4) und/oder der Verarbeitungseinrichtung (5) versehen ist.

8. Vorrichtung nach Anspruch 7 mit einer Verbindungseinrichtung (11, 12), die mit der Elektrodeneinrichtung (3, 4) und/oder mit der Verarbeitungseinrichtung (5) verbunden ist und zum Aufnehmen von jeweiligen komplementären Verbindungselementen (21, 22) der Kabeleinrichtung (15, 16) angeordnet ist.

9. Vorrichtung nach einem der Ansprüche 1 bis 8, wobei der Aufnahmekörper oder der Aufnahmerahmen ein Taschenformat aufweist und im Wesentlichen rechteckförmig mit einer Länge zwischen 70 und 100 mm, insbesondere von 85 mm, einer Breite zwischen 40 und 70, insbesondere von 55 mm, und einer Dicke zwischen 3 und 15 mm, insbesondere von 5 mm, ist.

10. Vorrichtung nach einem der vorangehenden Ansprüche mit einer Batterieeinrichtung (7) zum Speisen zumindest der Verarbeitungseinrichtung (5) und der Speichereinrichtung (6).

11. Vorrichtung nach Anspruch 10, wobei die Batterieeinrichtung entnehmbar ist.

12. Vorrichtung nach einem der vorangehenden Ansprüche mit einer Schaltereinrichtung (17) zum Aktivieren oder Deaktivieren zumindest der Verarbeitungseinrichtung (5).

13. Vorrichtung nach einem der vorangehenden Ansprüche, wobei die Verarbeitungseinrichtung (5) eine elektronische Einheit umfasst, die zum Umwandeln des elektrischen Signals vom analogen Typ, das durch die Elektrodeneinrichtung (3, 4) detektiert wird, in ein diskretes digitales Signal, das in Form einer Datei in der Speichereinrichtung (6) aufgezeichnet werden kann, angeordnet ist.

14. Vorrichtung nach einem der vorangehenden Ansprüche, wobei die Verarbeitungseinrichtung (5) zum Erfassen des elektrischen Signals mit einer vordefinierten Abtastfrequenz, insbesondere einer Frequenz von nicht weniger als 1000 Abtastwerten pro Sekunde, angeordnet ist.

15. Vorrichtung nach einem der vorangehenden Ansprüche, die zum Messen und Aufzeichnen des elektrischen Signals einer ECG-Kurve in einer D1-Standardleitung geeignet ist.

## Revendications

1. Appareil portable pour mesurer et stocker un signal électrique relatif à un tracé électrocardiographique, comprenant des moyens d'électrode (3, 4) pour interfacer des parties du corps d'un utilisateur pour détecter un signal électrique relatif à un tracé électrocardiographique de ce dernier, des moyens de traitement (5) pour acquérir ledit signal électrique grâce auxdits moyens d'électrode (3, 4), des moyens de mémoire (6) pour stocker ledit signal électrique acquis par lesdits moyens de traitement (5), lesdits moyens de mémoire (6) étant amovibles, des moyens de support ayant deux parois latérales (2a) et agencés pour contenir lesdits moyens de traitement (5) et lesdits moyens de mémoire (6), lesdits moyens d'électrode comprenant une première électrode (3) et une seconde électrode (4) connectées auxdits moyens de traitement (5) et fixées auxdits moyens de support (2) et insérées dans une partie desdits moyens de support (2) de sorte que chacune de ces électrodes peut être mise en contact avec au moins un doigt d'une main respective dudit utilisateur, **caractérisé en ce que** ladite première électrode (3) et ladite seconde électrode (4) sont formées et agencées de manière à présenter des surfaces d'interface libres respectives sur les deux parois latérales desdits moyens de support (2).

2. Appareil selon la revendication 1, dans lequel lesdits moyens de mémoire (6) comprennent au moins au moins une carte électronique à mémoire de masse non volatile.

3. Appareil selon la revendication 1, dans lequel lesdits moyens de mémoire (6) comprennent une «carte SIM» électronique programmable associable à un téléphone mobile pour connexion à un réseau de téléphonie mobile.

4. Appareil selon la revendication 1, dans lequel lesdits moyens de mémoire (6) comprennent une unité de stockage de masse portable utilisable au moyen d'un port USB.

5. Appareil selon l'une quelconque des revendications précédentes, comprenant des moyens d'interface (9) pour connecter lesdits moyens de traitement (5) auxdits moyens de mémoire (6).

6. Appareil selon l'une quelconque des revendications précédentes, comprenant des moyens de connexion (10) pour connecter lesdits moyens de mémoire (6) et/ou lesdits moyens de traitement (5) à des moyens de traitement électroniques externes, en particulier, à un ordinateur personnel.

7. Appareil selon l'une quelconque des revendications précédentes, comprenant en outre des moyens d'électrode (13, 14) dotés de moyens de câble (16, 15) pour une connexion amovible auxdits moyens d'électrode (3, 4) et/ou auxdits moyens de traitement (5).

8. Appareil selon la revendication 7, comprenant des moyens de liaison (11, 12) connectés auxdits moyens d'électrode (3, 4) et/ou auxdits moyens de traitement (5) et agencés pour recevoir des connecteurs complémentaires (21, 22) respectifs desdits moyens de câble (15, 16).

9. Appareil selon l'une quelconque des revendications 1 à 8, dans lequel ledit corps contenant ou ledit cadre contenant présente un format de poche et est de forme sensiblement rectangulaire, avec une longueur comprise entre 70 et 100 mm, en particulier de 85 mm, une largeur comprise entre 40 et 70 mm, en particulier de 55 mm, et une épaisseur comprise entre 3 et 15 mm, en particulier de 5 mm.

10. Appareil selon l'une quelconque des revendications précédentes, comprenant des moyens de batterie (7) pour alimenter au moins lesdits moyens de traitement (5) et lesdits moyens de mémoire (6).

11. Appareil selon la revendication 10, dans lequel lesdits moyens de batterie sont amovibles.

12. Appareil selon l'une quelconque des revendications précédentes, comprenant des moyens de commutation (17) pour activer ou désactiver au moins lesdits moyens de traitement (5).

13. Appareil selon l'une quelconque des revendications précédentes, dans lequel lesdits moyens de traitement (5) comprennent une unité électronique agencée pour convertir ledit signal électrique de type analogique, détecté par lesdits moyens d'électrode (3, 4) en un signal numérique discret qui peut être enregistré sous la forme d'un fichier de données sur lesdits moyens de mémoire (6).

14. Appareil selon l'une quelconque des revendications précédentes, dans lequel lesdits moyens de traitement (5) sont agencés pour acquérir ledit signal électrique avec une fréquence d'échantillonnage prédéfinie, en particulier, une fréquence non inférieure à 1000 échantillons par seconde.

15. Appareil selon l'une quelconque des revendications précédentes et approprié pour mesurer et enregistrer le signal électrique d'un tracé d'ECG sur la dérivation standard D1.
